Europäisches Patentamt

European Patent Office

Office européen des brevets

(11) Publication number : **0 621 044 A2**

# EUROPEAN PATENT APPLICATION

(21) Application number : **94302748.2**

(22) Date of filing : **18.04.94**

(51) Int. Cl.$^5$ : **A61L 27/00, A61L 15/32**

(30) Priority : **21.04.93 JP 116571/93**
**22.04.93 JP 117561/93**
**22.04.93 JP 117562/93**

(43) Date of publication of application :
**26.10.94 Bulletin 94/43**

(84) Designated Contracting States :
**DE FR GB IT**

(71) Applicant : **KABUSHIKI KAISHA SANGI**
**11-6, Tsukiji 3-chome,**
**Chuo-ku**
**Tokyo (JP)**

(72) Inventor : **Atsumi, Kiminori**
**c/o K.K. Sangi,**
**11-6 Tsukiji 3-chome,**
**Chuo-ku**
**Tokyo (JP)**

Inventor : **Saito, Tomoki**
**c/o K.K. Sangi,**
**11-6 Tsukiji 3-chome,**
**Chuo-ku**
**Tokyo (JP)**
Inventor : **Ishizaki, Tsutomu**
**c/o K.K. Sangi,**
**11-6 Tsukiji 3-chome,**
**Chuo-ku**
**Tokyo (JP)**
Inventor : **Fujita, Keijiro**
**c/o K.K. Sangi,**
**11-6 Tsukiji 3-chome,**
**Chuo-ku**
**Tokyo (JP)**
Inventor : **Kouyama, Hideo**
**c/o K.K. Sangi,**
**11-6 Tsukiji 3-chome,**
**Chuo-ku**
**Tokyo (JP)**

(74) Representative : **SERJEANTS**
**25, The Crescent**
**King Street**
**Leicester, LE1 6RX (GB)**

(54) **Collagen membranes.**

(57)    Collagen membranes containing inorganic and/or organic agents are useful for various medical treatments, e.g. as antibacterial artificial skin. The membranes, of high strength, are formed from collagen fibres obtained by refining and reconstituting collagen which has been treated by the limited pepsin digestion process. Suitable organic agents include bone forming agents as well as antibacterial agents, and suitable inorganic agents include calcium phosphate compound as well as antibacterial ceramic. The membranes can be laminated to air permeable membranes.

EP 0 621 044 A2

## BACKGROUND OF THE INVENTION

### Field of the Invention

The invention relates to collagen membranes containing inorganic agent and having high strength, in particular the invention relates to collagen membranes having high strength and being formed from collagen fibres obtained by refining and reconstituting collagen which have been treated by the limited pepsin digestion process and also this invention relates to a gradually releasing formulation, in which an organic agent is contained in the collagen membrane formed from collagen fibres obtained by refining and reconstituting collagen which has been treated by the limited pepsin digestion process. Suitable inorganic and/or organic agents include bone forming agents, antibacterial agents, calcium phosphate compound and antibacterial ceramic. The collagen membranes can be laminated to air permeable membranes.

### Prior Art

It is well known that collagen membranes can be used for medical treatment of conditions such as skin burns, defects of tooth bone, remineralization of tooth cement and bone fractures. However, when the collagen membranes are used, bacterial infections in the injury and burying portions, in which the collagen membranes are used, become the serious problems. Then during the medical treatments, the antibacterial agents and antibiotics are coated on the collagen membranes to prevent the bacteria infections, and as shown in the Japanese Patent Publication No. Sho 58-41054, the collagen membrane is used by immobilizing the antibiotics on the surface of collagen membrane. However, when the chemical agents are coated on the collagen membrane, the permeability of the chemical agent becomes a problem and when the antibiotics are immobilized on the collagen membrane, the problems, such as the effective time period of antibiotics and the tolerant bacteria, may occur.

Meantime, the collagen membranes are difficultly used by oneself due to a weak strength. The artificial skin is shown by the Japanese Patent Laid-Open Publication No. Hei 3-182262, in which the resin containing aluminosilicate containing silver is coated on the sheet suitable to living body, and also the method of using collagen membrane is taught by Japanese Patent Publication No. Sho 58-52662, in which collagen is adhered to an air permeable based material. However, the method disclosed in the former is a possible problem for safety, because the surface contacting the skin is the resin layer. Also, the experiments, by which the bones are introduced to the defect portions and the fractures of bone, are performed by using BMP which is the bone-inductive proteins, however, BMP is not effectively acted, because BMP is dispersed from the diseased parts with the body fluids.

The collagen membrance containing connective tissue such as bone inductive protein, hyaluronic acid, chondroitine suphate, fibronectin and osteronection is disclosed in Japanese Patent Laid-Open Publication Hei 2-156954 as the collagen membrane containing chemical agent.

Accordingly, the method for preventing the bacterial infection is effectively used, in which both the collagen membrane containing chemical agent and the silicon membrane are used, the silicon membrane is only peeled off and the coria of the patient are implanted to the peeling portion. However, this method is troublesome and the antigenic property of the collagen membrane cannot be avoided.

Collagens are widely distributed to the natural fields and exist mainly as the proteins constituting the animal bodies. Although collagens are originally less antigenic protein, it is not said that there has been no antigenic property at all. Accordingly, when used as artificial skin, it is naturally desirable to use the collagen membranes having high strength, non-antigenic property and homologous primary structure for human collagen.

## SUMMARY OF THE INVENTION

The collagen membranes used in the prior art have the problems shown in the above. Accordingly, the primary object of this invention is to provide the collagen membrane having high strength and containing various inorganic agents which can be used by oneself without using other membrane to increase the strength and the uneasiness for bacterial infection is not necessary to consider. The other object of this invention is to provide the collagen membrane containing organic and inorganic agents, in which the organic and inorganic agents are contained in the collagen membrane formed from collagen fibres obtained by refining and reconstituting collagen which has been treated by the pepsin digestion process.

Further the other object of this invention is to provide the collagen membrane having high strength and non-antigenic property and gradually releasing the organic agents contained in the collagen membrane to the diseased part.

2

## BRIEF DESCRIPTION OF THE DRAWINGS

Fig. 1 shows an electron microscope photograph of the strengthened collagen fibre membrane of the invention (x 100,000).

Fig. 2 shows an electron microscope photograph of the collagen membrane of the prior art (x 100,000).

## DETAILED DESCRIPTION OF THE INVENTION

To achieve the above-mentioned objects, the various tests were examined and it was recognized that the collagen membrane having high strength and not considering the bacterial infection is obtained by adding the inorganic agent such as calcium phosphate compound and antibacterial ceramic to the collagen membrane used in the prior art and/or the strengthened collagen fibre membrane obtained in performing the removing process of antigenic property for the collagen membrane. Also it was recognized that the collagen membrane having non-antigenic property and gradually releasing the organic agent is obtained by adding antibacterial agent, bone forming agent, the other organic agents which are used to accomplish the medical treatment for the objective disease and the mixture thereof, to the collagen membrane formed from collagen fibres obtained by refining and reconstituting collagen which has been treated by the limited pepsin digestion process.

The antigenic property of collagen mainly depends on the animal species and the antigen determining portions present in both the terminal portions of the collagen molecule. It is clear that both the terminal portions can be removed from the collagen molecule by using the pepsin limited digestion process for collagen. In accordance with this principle, he strengthened collagen fibre membrane to be used in the invention may be obtained by the method described below:

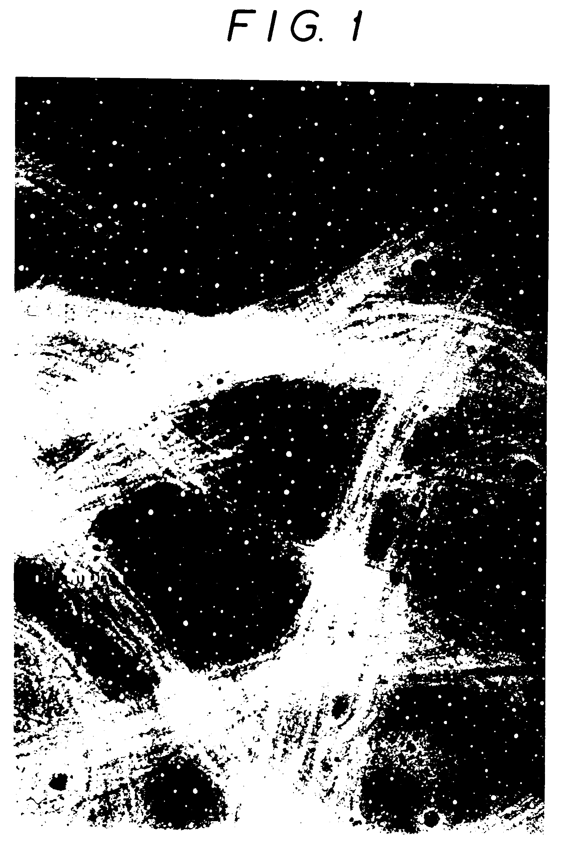

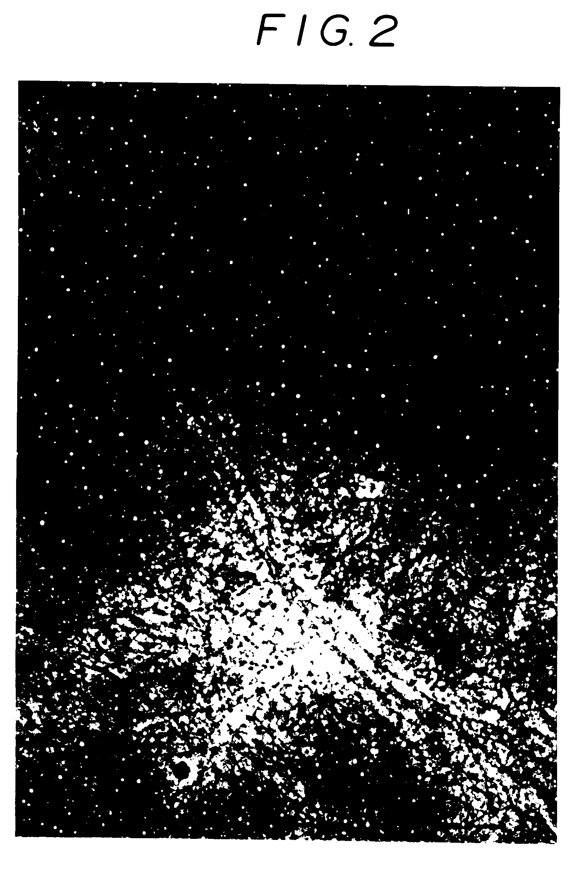

The pepsin limited digestion process is performed on collagen having a primary structure highly homologous with that of human collagen. Such a collagen is calf collagen. The antigen determining portions are thereby removed. The resultant collagens are refined and reconstituted to give collagens having thick fibres of 100 to 150 $\mu$m width (such thick fibre collagens are described hereinbelow as the strengthened collagen fibres). The strengthened collagen fibres are then bridged with a bridging agent. The strengthened collagen fibres obtained in this manner have the suitable thick diameter as shown in the natural form (see Fig. 1), display the physiological functions to the living body and have high strength, when compared with the commercial collagen membrane coagulating a fine fibre containing extremely fine width, about 10nm, (see Fig. 2) which is produced by heating the refined collagen at 37°C in buffer solution containing NaCl, gelling and freeze-drying.

The preparation of the strengthened collagen fibre membrane is exemplified by the following procedure.

### (1) The separation of the coria from the calf skin

The coria are separated by the usual method from the calf skin, crushed by the liquid nitrogen method and washed with phosphate buffer and NaCl aqueous solutions.

### (2) The pepsin digestion

The fine coria obtained as above described are solubilized with the pepsin limited digestion process and telopeptides having antigenic properties are mainly decomposed. The pepsin solubilized collagens are dialyzed and the collagens are precipitated.

### (3) The salt out refining

The collagens obtained by the pepsin digestion are dissolved in acetic acid solution. This solution is dialyzed in the acetic acid solution containing NaCl and the precipitated materials produced are separated. The collagen purity is increased by repeating this operation.

### (4) The reconstitution

The strengthened collagen fibres are obtained as the natural form fibre having thick fibre containing 100 to 150 $\mu$m width according to either of the following methods:

### Method 4-1:

The refined collagens are dissolved in acetic acid solution at about 1 % concentration and dialyzed in phosphate buffer solution having pH 7.4 and NaCl at low temperature, about 4°C. After the dialysis is finished, this solution is again dialyzed in $Na_2HPO_4$ solution at low temperature, about 4°C. Collagens are reconstituted to the thick fibre having natural form and washed with dilute $Na_2HPO_4$ solution.

Method 4-2:

The refined collagens are dissolved in 0.5M acetic acid solution at a concentration of 0.05 % and dialyzed in phosphate buffer solution having pH 7.4 and NaCl at about 4°C. Afterwards, this solution is dialyzed overnight at about 37°C. Collagens are reconstituted to the thick fibre having natural form and washed with dilute $Na_2HPO_4$ solution.

(5) The bridging

The strengthened collagen fibres are bridged according to either the following methods.

Method 5-1:

The bridge is introduced to the strengthened collagen fibre by reacting with hexamethyleneisothiocyanate at room temperature for 1 to 4 hours in 100 % ethanol solution. After the reaction, excess hexamethyleneisothiocyanate is removed by washing with 100 % ethanol.

Method 5-2:

The bridge is introduced by reacting the strengthened collagen fibre with glutaraldehyde at room temperature for 1 to 6 hours in neutral buffer solution. After the reaction, excess glutaraldehyde is removed by washing with distilled water.

(6) The hydration

When method 5-1 is used, distilled water is substituted for the ethanol.

(7) The freeze-drying

The freeze-drying is performed by entering the strengthened collagen fibres to a flame not to destroy the membrane structure of the collagen fibre membrane.

In this invention, the gelling collagens, the fibre collagens and the collagen membranes thereof which are used in the prior art, the strengthened collagen fibres and the strengthed collagen fibre membranes which are obtained in the above mentioned method can be used as collagen, particularly, the uses of strengthened collagen fibres and membranes thereof are preferable due to the high strength and non-antigenic property. Collagens are suspended in distilled water or buffer solution and the inorganic and/or organic agents are added to the suspension in order to obtain collagen containing inorganic and/or organic agent. It is possible to add the inorganic and/or organic agent to collagen in the range of from 0.01 to 100 parts of the inorganic and/or organic agent per 100 parts of collagen; the amount is most suitably selected according to the agent and the intended purpose. When the strengthened collagen fibre membrane is used as collagen and the activity of the inorganic and/or organic agent is not lost by bridging, it is possible to obtain the strengthened collagen fibre membrane containing inorganic and/or organic agent due to coexist the inorganic and/or organic agent with the bridging agent when bridging. However, as the activity of the inorganic and/or organic agent may be lost in the presence of bridging agent, it is preferable to obtain the strengthened collagen fibre membrane containing inorganic and/or organic agent after the formation of strengthened collagen fibre membrane. The collagen containing inorganic and/or organic agent is suspended in 70 % ethanol, filtered on a Buchner funnel having teflon cloth, compressed with a rod having a flat end and formed to the desired thickness. After that, washed with 100 % ethanol and it is difficult to obtain the collagen containing inorganic and/or organic agent except for using ethanol and teflon cloth, because the filtration cannot be performed because the filter is blocked.

All the organic agents may be used in this invention, particularly the skin permeable organic agents, such as anti-inflammation agents, hormones, antidiabetic agents, antiangina agents, antibiotics, antibacterial agents and antitumour agents, and bone forming proteins are preferable. Calcium phosphate compounds have good affinity to proteins and are easily carried on collagen; they further serve to strengthen the collagen membrane. Hydroxyapatite and calcium tertiary phosphate, the preferred calcium phosphate compounds, are useful as fillers for bone defects. The collagen membranes containing antibiotics and antibacterial agents formed from collagen fibres obtained by refining and reconstituting collagen which has been treated by the pepsin digestion process are useful for treating serious burns due both to their gradual release of the agents and their function as artificial skin. When the antibacterial ceramic, in which silver is carried on at least one ceramic selected from hydroxyapatite, calcium tertiary phosphate, zirconium phosphate, zeolite and zinc oxide, is used as the antibacterial agent, silver having antibacterial property is not eluted from the collagen membrane as silver ion, then a side effect of silver ion is not exhibited and the collagen membrane is strengthened with the antibacterial ceramic. Accordingly the collagen membrane containing antibacterial ceramic is useful as the antibacterial collagen membrane having high strength. Particularly, when hydroxyapatite or calcium tertiary phosphate is used, a good collagen membrane having safety and adhesive property to the skin and bone is obtained.

The antibacterial ceramic carrying silver may be prepared by a process in which a water soluble silver salt is carried on the ceramic with adsorption or ion exchange, after which the ceramic carrying silver is heat-fired at a temperature above the melting point of silver.

For example, the fine ceramic is added to the silver nitrate solution, mixed at ambient or elevated temperature, the ceramic is separated, washed with water, dried, heat-fired at the melting point of silver (960°C) and finely crushed. The combination of silver and ceramic is strengthened by heat-firing and the elution of silver from ceramic does not occur and the amounts of silver carried can be suitably selected by the concentration of silver salt to be used, the sort of ceramic, the treatment temperature and time period. However, when large amounts of silver are carried, the combination of silver and ceramic is prevented in some case and when the very small amounts of silver are carried, the antibacterial force becomes weak, so the amounts of silver carried on ceramic are from 0.0001 to 50 % by weight, preferably from 0.001 to about 20 % by weight with respect to ceramic.

The calcium phosphate compounds or antibacterial ceramics are preferably of fine particle size, e.g. less than 100 μm. More preferably, the particle size is less than 20 μm, the purpose is achieved by adding the small amounts of the particles and the collagen membrane having uniform dispersion is obtained. Also the amounts of calcium phosphate compound or antibacterial ceramics carried on the collagen membrane are suitably from 0.1 to 50 % by weight with respect to the collagen. Larger amounts would render the membrane hard and inflexible. Small amounts weaken the strength and the antibacterial property. Preferred amounts are from 0.5 to 30 % by weight with respect to the collagen.

It is also possible to add pigments, stabilizers, perfumes and the like to the collagen membranes according to the invention. It is possible to use the laminated or adhered collagen membrane, in which the collagen containing inorganic and/or organic agent is permeated or coated to an air permeable membrane or collagen membrane. Non-woven and textile fabrics made from natural fibres and from synthetic resins, such as cotton, pulp, paper, polyurethane, polyethylene and polypropylene, can be used as the air permeable membrane. The collagen membrane containing inorganic and/or organic agent laminated and adhered to the non-woven and/or textile fabrics is preferable in some cases due to the high strength and easy treatment.

The collagen membranes according to the invention are useful as gradual release formulations having high strength. They can be used by oneself and the amounts and the sort of inorganic and/or organic agent can be suitably selected according to the purpose. When the strengthened collagen fibre is used, the antigenic property and the harmful property for the living body are not developed, because the strengthened collagen fibre is the natural form fibre the same as the living body collagen. The following Examples illustrate the invention.

Reference Example

(1) The coria were prepared from calf skin by the usual method and crushed by the liquid nitrogen method. The fine coria obtained were washed with 0.2 M $Na_2HPO_4$ solution and 4.0M NaCl solution and solubilized by the limited digestion process with pepsin at 20°C.

Telopeptides having antigenic property were decomposed, and the pepsin solubilized collagens were dialyzed in 0.02M phosphate solution. Then the collagen precipitates were separated.

(2) The collagen precipitates were dissolved in 0.5M acetic acid solution. This was dialyzed in 0.5 M acetic acid solution containing 0.7M NaCl. The precipitates produced were separated. The refined collagen was obtained by repeating this process twice.

(3) The refined collagen was dissolved in 0.5M acetic acid solution at a concentration of 0.1 % and dialyzed in 0.01M phosphate buffer solution having pH7.4 and 0.15M NaCl at 4°C. After the dialysis was finished this solution was further dialyzed in 0.02M $Na_2HPO_4$ solution at 4°C and thick collagen fibres having natural form were obtained. These fibres were washed with 0.02M $Na_2HPO_4$ solution.

(4) The collagen fibres obtained were treated with 0.1 % glutaraldehyde in neutral buffer solution for 3 hours. The collagens obtained were washed with water, freeze-dried and the collagen fibre membranes were obtained. The electron microscope photograph thereof is shown in Fig. 1 and that of the prior art is shown in Fig. 2 for comparison.

(5) The collagen fibre membrane in this invention has a width of from 100 to 150 μm and the thick fibre having 700 nm periodic cross-striated structure characterized in collagen of the living body. The prior art collagen membrane has the imperfect fibre having 10 nm width and no cross-striated structure.

Example 1

Indomethacin and menthol were permeated to the strengthened collagen fibre membrane obtained in the above Reference Example at the rates of 10 mg/g and 30 mg/g respectively. This strengthened collagen fibre membrane 25 cm$^2$ was patched to a depilated back skin surface of a rabbit. After 6.24 and 24 hours, blood was collected from the ear vein of the rabbit and the concentrations of the agents in the blood plasma were determined. For comparison, indomethacin and menthol were permeated to the prior art collagen membrane at the rates of 10 mg/g and 30 mg/g respectively.

The results obtained are shown in Table 1.

Table 1

|  | indomethacin concentration in the blood plasma (ng/ml) | | |
|---|---|---|---|
|  | after 6 hours | after 24 hours | after 48 hours |
| Example | 59 | 48 | 41 |
| Comparison Example | 67 | 26 | 15 |

Example 2

The strengthened collagen fibre membrane was immersed in a solution containing BMP, and a strengthened collagen fibre membrane containing 0.2 % BMP was thereby obtained. This strengthened collagen fibre membrane was rolled around a fracture of a bone in a rat and the progress of healing was observed. For comparison, a prior art collagen fibre membrane containing 0.2 % BMP was also examined. When the strengthened collagen fibre membrane was used, the healing time was 3 weeks. When the prior art collagen membrane was used, the healing time was 5 weeks.

The results show that the strengthened collagen fibre membrane of this invention is a better gradual release carrier of chemical agents than the prior art collagen membrane.

Example 3

As mentioned above, the solubilized collagen obtained in the pepsin limited digestion process of the calf coria was refined, the strengthened collagen fibre having thick natural form was obtained and the strengthened collagen fibre membrane was obtained by bridging with glutaraldehyde.

The strengthened collagen fibre membrane was suspended in distilled water and calcium tertiary phosphate or hydroxyapatite having a particle size of under 20 μm was added under vigorous stirring at ambient temperature. The strengthened collagen fibre containing hydroxyapatite or calcium tertiary phosphate was obtained, then the strengthened collagen fibre membrane containing hydroxyapatite or calcium tertiary phosphate was obtained by treating with the method described follows (3-7 to 3-12).

Meanwhile, the prior art collagen membrane was suspended in distilled water and hydroxyapatite or calcium tertiary phosphate was added, vigorously stirred. The collagen membrane containing hydroxy-apatite or calcium tertiary phosphate was obtained by freeze-drying (3-1 to 3-6).

The contents of hydroxyapatite or calcium tertiary phosphate in the collagen membranes obtained are shown in Table 2. (Percentages in the Table are percentages by weight with respect to collagen).

Table 2

| | | chemical agent added | Amount added | collagen membrane |
|---|---|---|---|---|
| Examples | 3-1 | calcium tertiary phosphate | 0.1 % | collagen gel membrane |
| | 3-2 | hydroxyapatite | 0.5 % | collagen gel membrane |
| | 3-3 | hydroxyapatite | 1 % | collagen gel membrane |
| | 3-4 | calcium tertiary phosphate | 10 % | collagen gel membrane |
| | 3-5 | hydroxyapatite | 30 % | collagen gel membrane |
| | 3-6 | calcium tertiary phosphate | 50 % | collagen gel membrane |
| | 3-7 | hydroxyapatite | 0.1 % | strengthened collagen fiber membrane |
| | 3-8 | calcium tertiary phosphate | 0.5 % | strengthened collagen fiber membrane |
| | 3-9 | calcium tertiary phosphate | 1 % | strengthened collagen fiber membrane |
| | 3-10 | hydroxyapatite | 10 % | strengthened collagen fiber membrane |
| | 3-11 | calcium tertiary phosphate | 30 % | strengthened collagen fiber membrane |
| | 3-12 | hydroxyapatite | 50 % | strengthened collagen fiber membrane |
| Comparison | 3-1 | collagen gel membrane | - | |
| | 3-2 | strengthened collagen fiber membrane | - | |

## Example 4

The tensile strengths of the collagen membranes produced in Example 3 were measured and the results obtained are shown in Table 3. The results from the prior art collagen membranes are shown as comparison examples 3-1 and the result from the strengthened collagen fibre membranes are shown as the comparison example 3-2.

Table 3

| | Example | | | | | | Comparison Example |
|---|---|---|---|---|---|---|---|
| | 3-1 | 3-2 | 3-3 | 3-4 | 3-5 | 3-6 | 3-1 |
| tensile strength ($Kgf/mm^2$) | 0.8 | 1.3 | 1.7 | 1.7 | 1.9 | 1.9 | 0.2 |
| | Example | | | | | | Comparison Example |
| | 3-7 | 3-8 | 3-9 | 3-10 | 3-11 | 3-12 | 3-2 |
| tensile strength ($Kgf/mm^2$) | 1.7 | 1.8 | 2.1 | 2.6 | 2.4 | 2.5 | 1.2 |

It is clear from Table 3 that the tensile strength of the collagen membrane is increased by adding calcium tertiary phosphate or hydroxyapatite to the collagen membrane; that the addition of hydroxyapatite is better than that of calcium tertiary phosphate; and that adding large amounts does not further improve the tensile strength. Also it is felt that when hydroxyapatite is added to the strengthened collagen fibre membrane in an amount of 50 %, the flexibility of the membrane is decreased and the tensile strength is reduced.

Example 5

Calcium tertiary phosphate carrying 2 % of silver was added to the strengthened collagen fibres and an antibacterial collagen membrane containing calcium tertiary phosphate carrying 1 % of silver was obtained by freeze-drying.

Example 6

Zinc oxide carrying 3 % of silver was added to the strengthened collagen fibre and an antibacterial collagen fibre containing zinc oxide carrying 1 % of silver was obtained by freeze-drying.

Example 7

Collagen containing 20 % of hydroxyapatite carrying 0.1 % of silver was coated to the strengthened collagen fibre membrane and the antibacterial strengthened collagen fibre membrane laminated was obtained by freeze-drying.

Example 8

Zeolite carrying 5 % of silver was added to the collagen gel, then the collagen gel obtained was coated to the cotton cloth.

Example 9

Collagen containing 20 % of zirconium phosphate carrying 1 % of silver was coated to polyurethane film and the antibacterial collagen membrane was obtained.

Example 10

The collagen membranes obtained in Examples 5 to 9 were cut to samples sized 5 x 4 cm and the samples were incubated in 10 ml of a solution containing Staphylococcus aureus, $1 \times 10^5$/ml, for 24 hours. The number of bacteria was calculated. For comparison, a similar test was carried out on a collagen membrane containing no antibacterial ceramic (Comparison Example 1).

The results obtained are shown in Table 4.

Table 4

| Sample | viable bacteria numbers after 24 hours/ml |
|---|---|
| Example 5 | 0 |
| Example 6 | 0 |
| Example 7 | 0 |
| Example 8 | 0 |
| Example 9 | 0 |
| Comparison Example 1 | $8 \times 10^4$ |

Example 11

The progress of healing was observed by rolling the collagen membranes obtained in Examples 5 and 6 around a fractured rat leg bone. A similar test was carried out for comparison, using a collagen membrane containing no antibacterial ceramic (Comparison Example 2). The healing time period was 4 weeks using the collagen membrane of Example 5, 3.5 weeks using the collagen membrane of Example 6 and 5.5 weeks in the comparison test.

Example 12

The collagen membranes obtained in Examples 7 to 9 having the same area as a wound were closely adhered to the whole layer defective wound (1 x 1 cm) in the back skin of SD rat, which is produced surgically, with a mesh type tape and the progress of healing was observed. A collagen membrane containing no antibacterial ceramic was used for a comparison test (Comparison Example 3).

The results are shown in Table 5.

Table 5

| | after 3 days | after 7 days | after 8 days |
|---|---|---|---|
| Example 7 | slight granulation | granulation | healing |
| Example 8 | juvenile granulation | slight granulation | decreased wound, roughly drying |
| Example 9 | juvenile granulation | slight granulation | decreased wound, drying |
| Comparison Example 3 | bleeding in the diseased part | juvenile granulation | bleeding in the diseased part, when the collagen membrane is peeled |

Claims

1.    A collagen membrane containing an inorganic agent

2. A collagen membrane according to claim 1, wherein the inorganic agent is a calcium phosphate based compound.

3. A collagen membrane according to claim 1, wherein the amount of the calcium phosphate based compound is in the range of from 0.1 to 50 % by weight with respect to the amount of collagen used.

4. A collagen membrane according to claim 2 or 3, wherein the calcium phosphate based compound is hydroxyapatite or calcium tertiary phosphate.

5. A collagen membrane according to Claim 1, wherein the inorganic agent is an antibacterial ceramic.

6. A collagen membrane according to claim 5, wherein the antibacterial ceramic is ceramic carrying silver.

7. A collagen membrane according to claim 5 or 6, wherein the ceramic is hydroxy apatite, calcium tertiary phosphate, zirconium phosphate, zeolite or zinc oxide.

8. A gradually releasing formulation of an organic agent, wherein an organic agent is contained in the collagen membrane formed from collagen fibres obtained by refining and reconstituting collagen which has been treated by the limited pepsin digestion process.

9. A gradually releasing formulation according to claim 8, wherein the organic agent is an antibacterial agent.

10. A gradually releasing formulation according to claim 8, wherein the organic agent is a bone forming protein.

11. A gradually releasing formulation according to claim 10, wherein the bone forming protein is Bone Marphozenic Protein.

12. A collagen membrane containing an inorganic agent, wherein the collagen membrane is formed from collagen fibres obtained by refining and reconstituting collagen which has been treated by the limited pepsin digestion process.

13. A collagen membrane according to claim 12, wherein the inorganic agent is a calcium phosphate based compound.

14. A collagen membrane according to claim 13, wherein the amount of the calcium phosphate based compound is in the range of from 0.1 to 50 % by weight with respect to the amount of collagen used.

15. A collagen membrane according to claim 13 or 14, wherein the calcium phosphate based compound is hydroxyapatite or calcium tertiary phosphate.

16. A collagen membrane according to claim 12, wherein the inorganic agent is an antibacterial ceramic.

17. A collagen membrane according to claim 16, wherein the antibacterial ceramic is ceramic carrying silver.

18. A collagen membrane according to claim 16 or 17, wherein the ceramic is hydroxyapatite, calcium tertiary phosphate, zirconium phosphate, zeolite or zinc oxide.

19. A collagen membrane according to any preceding claim adhered and/or laminated to a collagen membrane.

20. A collagen membrane according to any preceding claim permeated and/or coated to an air permeable membrane.

21. A collagen membrane according to claim 20 wherein the air permeable membrane is selected from a natural fibre such as cotton fibre, pulp and paper and non-woven fabric textile fabric made from a synthetic resin.

# F I G. 1

# FIG. 2